Europäisches Patentamt

European Patent Office

Office européen des brevets

(19)

(11) Veröffentlichungsnummer: **0 014 392**
**B1**

(12) **EUROPÄISCHE PATENTSCHRIFT**

(45) Veröffentlichungstag der Patentschrift:
04.11.81

(21) Anmeldenummer: 80100385.6

(22) Anmeldetag: 25.01.80

(51) Int. Cl.³: **A 61 K 31/505** // (A61K31/505, 31/415)

(54) Antithrombotische Arzneimittelkombination und Verfahren zu ihrer Herstellung.

(30) Priorität: 08.02.79 DE 2904736

(43) Veröffentlichungstag der Anmeldung:
20.08.80 Patentblatt 80/17

(45) Bekanntmachung des Hinweises auf die Patenterteilung:
04.11.81 Patentblatt 81/44

(84) Benannte Vertragsstaaten.
BE CH DE FR GB IT LU NL SE

(56) Entgegenhaltungen:
EP-A-0 000 774
US-A-3 322 755
CHEMICAL ABSTRACTS, Band 75,
Nr. 9, 30. August 1971, Seite 289, Nr. 61768b
Columbus, Ohio, U.S.A.
P. DIDISHEIM et al.: »Effect of dipyridamole (Persantin) and its derivatives on thrombosis and platelet function«
CHEMICAL ABSTRACTS, Band 89,
Nr. 9, 28. August 1978, Seite 47, Nr. 70986n
Columbus, Ohio, U.S.A.
J. L. AMBRUS et al.: »Studies on platelet aggregation in vivo. VI. Effect of a pyrimido-pyrimidine derivative (RA 233) on tumor cell metastasis«

(73) Patentinhaber: Dr. Karl Thomae GmbH, Postfach 720, D-7950 Biberach (Riss) (DE)

(72) Erfinder: Slichter, Sherrill J., Dr., 5201 S.W. Canada Dr., Seattle/Washington 98126 (US)

## Antithrombotische Arzneimittelkombination und Verfahren zu ihrer Herstellung

Gegenstand der vorliegenden Anmeldung ist eine neue synergistisch wirkende antithrombotische Arzneimittelkombination, enthaltend 2,6-Bis(diäthanolamino)-4-piperidino-pyrimido[5,4-d]pyrimidin (siehe US-PS 3 322 755) und 1,2-Diphenyl-3,5-dioxo-4-(2-phenylsulfinyläthyl)-pyrazolidin (generic name: Sulfinpyrazon — siehe Helv. chim. Acta 44, 336 (1961)) neben gegebenenfalls einem oder mehreren inerten Trägerstoffen und/oder Verdünnungsmitteln.

Die antithrombotische Wirkung beider Substanzen ist literaturbekannt (siehe beispielsweise Therapiewoche 26, 8464—8489 (1976)). Bei beiden Substanzen ist jedoch zur Erzielung einer antithrombotischen Wirkung eine relativ hohe Dosis erforderlich, wobei sich schon teilweise die Nebenwirkungen beider Substanzen bemerkbar machen, beispielsweise bei 1,2-Diphenyl-3,5-dioxo-4-(2-phenylsulfinyläthyl)-pyrazolidin sind es Magenschmerzen wegen der in hohen Dosen ulcerogenen Wirkung dieser Substanz.

Überraschenderweise wurde nun eine stark synergistische Wirkung der beiden Wirkstoffe festgestellt. Bei einer Kombination dieser beiden Substanzen können die erforderlichen Dosen zur Erreichung des gleichen antithrombotischen Effektes der jeweiligen Einzelsubstanz wesentlich gesenkt werden. Diese synergistische Wirkung wurde am folgenden Versuchsmodell festgestellt:

Tiere, die an bösartigen Tumoren erkrankt sind, haben einen erhöhten Umsatz an Gerinnungsfaktoren und an Thrombozyten. Der Thrombozytenumsatz kann leicht mittels radioaktiv markierter Thrombozyten gemessen werden. Hierbei wird den Versuchstieren Blut entnommen, die Erythrozyten werden abgetrennt und dem plättchenreichen Plasma wird eine geeignete Menge Chrom-51 zugesetzt, die sich in den Thrombozyten anreichert. Das überflüssige, im Plasma befindliche Chrom-51 wird abzentrifugiert und verworfen. Das Thrombozytenkonzentrat mit den markierten Zellen wird nun dem Tier i. v. injiziert. Zu bestimmten Zeitpunkten werden Blutproben entnommen und deren Gehalt an Radioaktivität bestimmt. Die Abnahme der Aktivität im Verlauf der Zeit, zweckmäßigerweise während 5 Tagen, wird zur Beurteilung der Thrombozytenlebenszeit herangezogen.

Die normale Thrombozytenlebenszeit beträgt bei Tieren, z. B. Hunden, etwa 5 Tage. Sie wird durch verschiedene Krankheiten verkürzt (bei bösartigen Tumoren z. B. bis auf etwa 2 Tage).

Bei der Applikation der erfindungsgemäßen Kombination (2,6-Bis-(diäthanolamino)-4-piperidino-pyrimido[5,4-d]pyrimidin/1,2-Diphenyl-3,5-dioxo-4-(2-phenylsulfinyläthyl)-pyrazolidin im Verhältnis 1 : 1 bis 1 : 2) an an Tumor erkrankte Hunde in einer Dosis von 50 bis 100 mg/kg wurde eine annähernd normale Thrombozytenlebenszeit von 4,5 bis 4,8 Tagen gefunden.

Die Normalisierung der Thrombozytenlebenszeit ist therapeutisch von sehr großer Bedeutung, da die Verkürzung der Thrombozytenlebenszeit ein Indiz für die Neigung zu Thrombosen ist. Die neue Arzneimittelkombination ist daher zur Vorbeugung und Heilung von thromboembolischen Krankheiten beim Menschen gut geeignet, außerdem hemmt sie die Fixierung von in die Blutbahn geschwemmten Krebszellen.

Die Einzeldosis für Erwachsene beträgt zwischen 25 und 200 mg der beiden Wirkstoffe, vorzugsweise 25 bis 100 mg 2,6-Bis(diäthanolamino)-4-piperidino-pyrimido[5,4-d]pyrimidin und 50 bis 175 mg 1,2-Diphenyl-3,5-dioxo-4-(2-phenylsulfinyl)-pyrazolidin, 2—4 × täglich.

Die neue Kombination weist eine sehr gute Verträglichkeit auf. So wurden beispielsweise an der Maus bei einer Dosis von 100 mg/kg p. o. der erfindungsgemäßen Kombination im Verhältnis 1 : 1 bis 1 : 2 der beiden Wirkstoffe keine toxischen Nebenwirkungen festgestellt. Die $LD_{50}$ an der Maus beträgt für 2,6-Bis(diäthanolamino)-4-piperidino-pyrimido[5,4-d]pyrimidin

465 mg/kg p. o. und
148 mg/kg i. v.

bzw. für 1,2-Diphenyl-3,5-dioxo-4-(2-phenylsulfinyläthyl)-pyrazolidin

298 mg/kg p. o. und
240 mg/kg i. v.

Gegenstand der Erfindung ist des weiteren ein Verfahren zur Herstellung der erfindungsgemäßen Arzneimittelkombination, welches dadurch gekennzeichnet ist, daß 2,6-Bis(diäthanolamino)-4-piperidino-pyrimido[5,4-d]pyrimidin und 1,2-Diphenyl-3,5-dioxo-4-(2-phenylsulfinyläthyl)-pyrazolidin im Verhältnis von 10 : 1 bis 1 : 10 kombiniert und gegebenenfalls mit den bei der Herstellung von Arzneimitteln üblichen Träger- und/oder Hilfsstoffen zu Tabletten, Dragees, Pulver für Briefe, Kapseln und dergleichen formuliert wird. Die erfindungsgemäßen neuen Präparate werden vorzugsweise peroral in den oben angeführten Dosen verabreicht.

Die Herstellung von Tabletten, Dragees, Kapseln etc. erfolgt in an sich bekannter Weise, beispielsweise werden die Tabletten durch unmittelbares Verpressen eines Gemisches der Wirk- und Hilfsstoffe hergestellt und gegebenenfalls nachträglich mit einem im Magen und Darm verträglichen Film überzogen, bei der Herstellung der Kapseln wird erst die Pulvermischung und der Kern mit Überzug getrennt hergestellt und dann auf einer handelsüblichen Kapselabfüllmaschine abgefüllt.
Die folgenden Beispiele erläutern die Erfindung ohne sie zu beschränken:

### Beispiel 1

#### Dragees mit 50 mg 2,6-Bis(diäthanolamino)-4-piperidono-pyrimido-[5,4-d]pyrimidin und 50 mg Sulfinpyrazon

Ein Drageekern enthält:

| | |
|---|---|
| 2,6-Bis(diäthanolamino)-4-piperidino-pyrimido[5,4-d]pyrimidin | 50,0 mg |
| Sulfinpyrazon | 50,0 mg |
| Milchzucker | 187,0 mg |
| Maisstärke | 105,0 mg |
| Gelatine | 6,0 mg |
| Magnesiumstearat | 2,0 mg |
| | 400,0 mg |

Die Wirkstoffe werden zusammen mit Milchzucker und Stärke gemischt und die Mischung gleichmäßig mit einer wäßrigen Gelatine-Lösung befeuchtet. Die feuchte Masse wird auf max. 2 mm gesiebt, getrocknet und nach nochmaligem Sieben mit dem Schmiermittel vermischt. Aus der so hergestellten preßfertigen Mischung wurden Tabletten von 400 mg gepreßt.

Drageekernmaße:

| | |
|---|---|
| Gewicht: | ca. 400 mg |
| Form | rund, bikonvex |
| Durchmesser | 11 mm |

### Dragierung

Die Kerne werden mit einer üblichen Zuckerdragiersuspension auf 500 mg und anschließend mit Zuckersirup auf 530 mg/Dragee dragiert.

### Beispiel 2

#### Dragèes mit 25 mg 2,6-Bis(diäthanolamino)-4-piperidino-pyrimido-[5,4-d]pyrimidin und 50 mg Sulfinpyrazon

Ein Dragéekern enthält:

| | |
|---|---|
| 2,6-Bis(diäthanolamino)-4-piperidino-pyrimido[5,4-d]pyrimidin | 25,0 mg |
| Sulfinpyrazon | 50,0 mg |
| Milchzucker | 187,0 mg |
| Maisstärke | 130,0 mg |
| Gelatine | 6,0 mg |
| Magnesiumstearat | 2,0 mg |
| | 400,0 mg |

Die Wirkstoffe werden zusammen mit Milchzucker und Stärke gemischt und die Mischung gleichmäßig mit einer wäßrigen Gelatinelösung befeuchtet. Die feuchte Masse wird auf max. 2 mm gesiebt, getrocknet und nach nochmaligem Sieben mit dem Schmiermittel vermischt. Aus der so hergestellten preßfertigen Mischung wurden Tabletten von 400 mg gepreßt

Dragèekernmaße:

| | |
|---|---|
| Gewicht: | ca. 400 mg |
| Form | rund, bikonvex |
| Durchmesser | 11 mm |

**0 014 392**

## Dragierung

Die Kerne werden mit einer üblichen Zuckerdragiersuspension auf 500 mg und anschließend mit Zuckersirup auf 530 mg/Dragée dragiert.

## Beispiel 3

Kapseln mit 50 mg 2,6-Bis(diäthanolamino)-
4-piperidino-pyrimido-[5,4-d]pyrimidin und 50 mg Sulfinpyrazon

Kapselhülle: Hartgelatine-Kapsel Größe 0

Kapselinhaltsstoffe:
Pulvermischung:

| | | |
|---|---|---|
| Sulfinpyrazon | | 50,0 mg |
| Maisstärke | ca. | 158,0 mg |
| Milchzucker pulv. | ca. | 90,0 mg |
| Magnesiumstearat | | 2,0 mg |
| | | 300,0 mg |

Kern ∅ 6 mm:

| | |
|---|---|
| 2,6-Bis(diäthanolamino)-4-piperidino-pyrimido[5,4-d]pyrimidin | 50,0 mg |
| Kollidon 25R (Polyvinylpyrrolidon) | 2,5 mg |
| Formaldehydgelatine | 6,5 mg |
| Magnesiumstearat | 1,0 mg |
| Milchzucker | 25,0 mg |

Überzug:

| | | |
|---|---|---|
| Talkum | ca. | 23,4 mg |
| Zucker | ca. | 4,2 mg |
| Gummi arabicum | ca. | 2,4 mg |
| | ca. | 105,0 mg |

Gesamtfüllgewicht: Pulver/Kern ca. 405 mg.

## Patentansprüche

1. Antithrombotische Arzneimittelkombination, dadurch gekennzeichnet, daß sie als Wirkstoffe 2,6-Bis(diäthanolamino)-4-piperidino-pyrimido-[5,4-d]pyrimidin und 1,2-Diphenyl-3,5-dioxo-4-(2-phenylsulfinyläthyl)-pyrazolidin neben üblichen Träger- und/oder Hilfsstoffen enthält.

2. Arzneimittelkombination nach Anspruch 1, dadurch gekennzeichnet, daß das Gewichtsverhältnis von 2,6-Bis(diäthanolamino)-4-piperidino-pyrimido[5,4-d]pyrimidin zu 1,2-Diphenyl-3,5-dioxo-4-(2-phenylsulfinyläthyl)-pyrazolidin 10 : 1 bis 1 : 10 beträgt.

3. Arzneimittelkombination gemäß Anspruch 1 und 2, dadurch gekennzeichnet, daß das Verhältnis der beiden Wirkstoffe 1 : 1 bis 1 : 2 beträgt.

4. Verfahren zur Herstellung einer antithrombotischen Arzneimittelkombination, dadurch gekennzeichnet, daß man 2,6-Bis(diäthanolamino)-4-piperidino-pyrimido[5,4-d]pyrimidin und 1,2-Diphenyl-3,5-dioxo-4-(2-phenylsulfinyläthyl)-pyrazolidin im Verhältnis von 10 : 1 bis 1 : 10 kombiniert und zusammen mit den üblichen Träger- und/oder Hilfsstoffen zu Dragées, Tabletten, Kapseln oder Pulverbriefchen formuliert.

## Claims

1. Antithrombotic drug combination, characterised in that it contains as active ingredients 2,6-bis(diethanolamino)-4-piperidino-pyrimido-[5,4-d]pyrimidine and 1,2-diphenyl-3,5-dioxo-4-(2-phenylsulphinylethyl)-pyrazolidine together with conventional carriers and/or excipients.

2. Drug combination as claimed in claim 1, characterised in that the weight ratio of 2,6-bis(diethanolamino)-4-piperidino-pyrimido[5,4-d]pyrimidine to 1,2-diphenyl-3,5-dioxo-4-(2-phenylsulphinylethyl)-pyrazolidine is from 10 : 1 to 1 : 10.

4

3. Drug combination as claimed in claims 1 and 2, characterised in that the ratio of the two active ingredients is from 1 : 1 to 1 : 2.

4. Process for the preparation of an antithrombotic drug combination, characterised in that 2,6-bis(diethanolamino)-4-piperidino-pyrimido[5,4-d]pyrimidine and 1,2-diphenyl-3,5-dioxo-4-(2-phenylsulphinylethyl)-pyrazolidine are combined in a ratio of 10 : 1 to 1 . 10 and formulated with conventional carriers and/or excipients to form coated tablets, tablets, capsules or sachets of powder.

**Revendications**

1. Combinaison médicamenteuse antithrombotique, caractérisée en ce qu'elle contient, en tant que substances actives, de la 2,6-bis(diéthanolamino)-4-pipéridino-pyrimido-[5,4-d]pyrimidine et de la 1,2-diphényl-3,5-dioxo-4-(2-phénylsulfinyléthyl)-pyrazolidine, avec des excipients et/ou adjuvants habituels.

2. Combinaison médicamenteuse selon la revendication 1, caractérisée en ce que le rapport en poids de la 2,6-bis(diéthanolamino)-4-pipéridino-pyrimido[5,4-d]pyrimidine à la 1,2-diphényl-3,5-dioxo-4-(2-phénylsulfinyléthyl)-pyrazolidine est de 10 : 1 à 1 : 10.

3. Combinaison médicamenteuse selon la revendication 1 et 2, caractérisée en ce que le rapport des deux substances actives est de 1 : 1 à 1 : 2.

4. Procédé pour la préparation d'une combinaison médicamenteuse antithrombotique, caractérisé en ce que l'on combine de la 2,6-bis(diéthanolamino)-4-pipéridino-pyrimido[5,4-d]pyrimidine et de la 1,2-diphényl-3,5-dioxo-4-(2-phénylsulfinyléthyl)-pyrazolidine dans le rapport de 10 : 1 à 1 : 10 et on les formule ensemble en dragées, comprimés, capsules ou sachets de poudre, avec les excipients et/ou adjuvants habituels.